# EUROPÄISCHE PATENTANMELDUNG

(11) **EP 3 730 485 A1**
(43) Veröffentlichungstag der Anmeldung: **28.10.2020**
(21) Anmeldenummer: 19171088.8
(22) Anmeldetag: 25.04.2019
(51) Int. Cl.: C07D 277/80

(54) **VERFAHREN ZUR HERSTELLUNG VON N,N-DISUBSTITUIERTEN BENZTHIAZOLYL-SULFENAMIDEN**

(71) Anmelder: LANXESS Deutschland GmbH, 50569 Köln (DE)
(72) Erfinder: GRAF, Holger, 42781 Haan (DE); WIEDEMEIER-JARAD, Melanie, 41540 Dormagen (DE); DE SMET, Nele, 9320 Nieuwerkerken (BE); HAGEMANN, Jörg, 51061 Köln (DE)

(57) **Zusammenfassung**

Die Erfindung betrifft ein Verfahren zur Herstellung von N,N-disubstituierten Benzthiazolylsulfenamiden.

## Beschreibung

Die Erfindung betrifft ein Verfahren zur Herstellung von N,N-disubstituierten Benzthiazolylsulfenamiden.

N,N-disubstituierten Benzthiazolyl-sulfenamide werden vor allem als langsam wirkende Vulkanisationsbeschleuniger in der Kautschukindustrie eingesetzt, insbesondere in der Reifenindustrie in Haftmischungen.

Verfahren zur Herstellung von N,N-disubstituierten Benzthiazolyl-sulfenamiden sind z.B. aus EP-A-0 721 946 bekannt. In einer Eintopfsynthese werden Dibenzthiazolyldisulfid, Dibenzylamin und Methanol vorgelegt und auf 60°C erwärmt, bevor Chlor über ein Kapillarrohr eingeleitet wird. Nach der anschließenden Behandlung mit 25%iger Natronlauge wird das Produkt abfiltriert und mit Methanol und Wasser gewaschen.

Chlor / Chlorgas ist dafür bekannt, dass es ein sehr gut wirkendes Oxidationsmittel ist. Im Umgang mit dieser hochgiftigen Chemikalien sind allerdings besondere Sicherheitsmaßnahmen einzuhalten. Dabei sind alle denkbaren Risiken bei Produktion, Transport sowie Lagerung aufgrund von Störfällen und Unfällen zu berücksichtigen. Ausbrüche von Chlor- und Chlorwasserstoffgas in die Atmosphäre gilt es auf jeden Fall zu verhindern, so dass nach einer Alternative für das vorgenannte Herstellverfahren gesucht wurde.

Aufgabe der vorliegenden Erfindung war es nun, ein wirtschaftliches und leichter handhabbares Verfahren zur Herstellung von N,N-disubstituierten Benzthiazol-sulfenamiden, insbesondere N,N-dibenzyl-benzthiazolylsulfenamid (DBBS) oder N,N-Diisopropylbenzthiazolyl-sulfenamid, ausgehend von substituierten 2-Mercaptobenzthiazolen unter Berücksichtigung sicherheitstechnischer Aspekte, vorzugweise in einer Eintopfreaktion, zur Verfügung zu stellen. Dabei sollte insbesondere ein anderes Oxidationsmittel als Chlorgas eingesetzt werden, um die Risiken mit dem Umgang dieser Chemikalie zu minimieren.

Überraschenderweise wurde nun gefunden, dass sich N,N-disubstituierten Benzthiazolsulfenamide durch die Umsetzung von substituierten 2-Mercaptobenzthiazolen mit Aminen in Gegenwart von Natriumhypochlorit als Oxidationsmittel herstellen lassen.

Gegenstand der vorliegenden Erfindung ist ein Verfahren zur Herstellung von N,N-disubstituierten Benzthiazolyl-sulfenamiden der Formel (I), 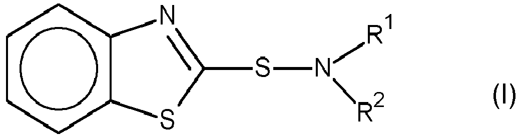
mit R¹, R² = unabhängig voneinander -CH(R³)(R⁴)
mit R³, R⁴ = unabhängig voneinander H, C₁C₃-Alkyl, vorzugsweise Methyl, oder 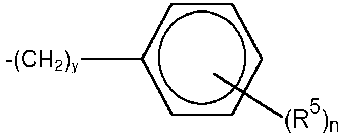
mit R⁵ = C₁-C₃-Alkyl,
y = 0 bis 6, vorzugsweise 0 bis 2, besonders bevorzugt 0 und
n = 0 bis 5, vorzugsweise 0,
durch Umsetzung von 2-Mercaptobenzthiazolsalzen der Formel (II) 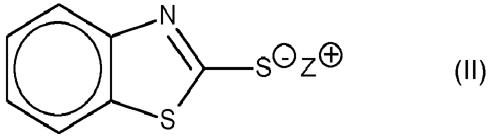 mit Z = Na oder K, vorzugsweise Na,
mit Aminen der Formel (III)

NH(R¹)(R²) (III)

worin R¹ und R² die obige Bedeutung haben,
wonach die Umsetzung in Gegenwart von Natriumhypochlorit durchgeführt wird.

Vorzugsweise handelt es sich bei der Verbindung der Formel (I) um N,N-Dibenzylbenzthiazolylsulfenamid (DBBS) oder um N,N-Diisopropylbenzthiazolyl-sulfenamid.

In einer bevorzugten Ausführungsform des erfindungsgemäßen Verfahrens sind die Reste R¹, R² innerhalb des Moleküls gleich.

Als Verbindungen der Formel (II) ist das Natriumsalz des 2-Mercaptobenzothiazols (NaMBT) bevorzugt.

Als Verbindungen der Formel (III) sind 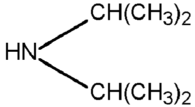 und 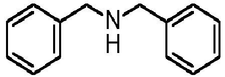 bevorzugt.

In einer weiteren bevorzugten Ausführungsform des erfindungsgemäßen Verfahrens wird als Verbindung der Formel (II) 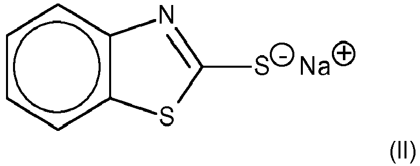 in Kombination mit 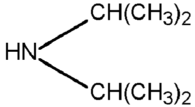 oder 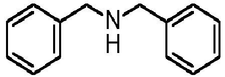 als Verbindung der Formel (III) eingesetzt.

Als Natriumhypochlorit wird vorzugsweise eine mindestens 14%ige Natriumhypochloritlösung eingesetzt. Besonders bevorzugt sind 14,1 - 18% ige Natriumhypochloritlösungen.

In einer bevorzugten Ausführungsform der Erfindung wird das Verfahren bei Temperaturen von 30 bis 50 °C, besonders bevorzugt bei 40 bis 50°C, durchgeführt.

In einer bevorzugten Ausführungsform der Erfindung wird das Verfahren in Anwesenheit von C₁-C₄-Alkohlen und/oder Wasser durchgeführt.

Als C₁-C₄-Alkohole im Sinne der Erfindung sind Methanol, Ethanol, Isopropanol, n-Butanol, tert.-Butanol oder Gemische dieser Alkohole bevorzugt. Besonders bevorzugt wird in dem erfindungsgemäßen Verfahren Isopropanol eingesetzt.

Vorzugsweise wird in dem erfindungsgemäßen Verfahren die Verbindung der Formel (III) gegebenenfalls in Wasser und/oder Alkohol vorgelegt und die Verbindung der Formel (II) und Natriumhypochloritlösung zudosiert.

In einer weiteren bevorzugten Ausführungsform der Erfindung wird das Verfahren bei pH-Werten von 10 bis 12 (bestimmt bei 46°C) durchgeführt. Dabei ist insbesondere bevorzugt ein pH-Wert im Bereich von 10,3 - 11. Zum Einstellen des pH-Wertes wird vorzugsweise eine Säure zu dosiert, vorzugsweise während der Zugabe der Verbindung (II) und dem Natriumhypochlorit. Als Säure bevorzugt sind starke anorganische Säuren, besonders bevorzugt Schwefelsäure.

In einer weiteren bevorzugten Ausführungsform der Erfindung wird der pH-Wert nach Zugabe der Verbindung der Formel (II) mit Natronlauge eingestellt.

Sofern das erfindungsgemäße Verfahren in Anwesenheit von Alkohol durchgeführt wird, werden bevorzugt auf 50-1000 Teile Amin der Formel (II), besonders bevorzugt N,N,-Diphenylamin, 300 bis 1300 Teile Alkohol (100%) eingesetzt. Besonders bevorzugt werden darauf noch zusätzlich 50-1000 Teile Wasser hinzugegeben.

Bei dem erfindungsgemäßen Verfahren wird vorzugsweise die Verbindung Formel (II) zu der Verbindung der Formel (III) in einem molaren Verhältnis von 1,4 : 1 bis 0,8 : 1 eingesetzt.

Bevorzugt wird das erfindungsgemäße Verfahren in diskontinuierlicher Fahrweise, in einer sogenannten Eintopfreaktion, durchgeführt.

Für das erfindungsgemäße Verfahren liegt die Reaktionszeit vorzugsweise im Bereich von 15 Minuten bis 5 Stunden, ganz besonders bevorzugt 2-5 Stunden.

Die Zugabe der Natriumhypochlorits erfolgt üblicherweise so, dass man dieses über eine Dosierpumpe und unter Rühren einleitet.

Die Abtrennung der Verbindung der Formel (I) erfolgt vorzugsweise mittels Filtration, wobei übliche Filternutschen geeignet sind.

In einer weiteren Ausführungsform der Erfindung wird das Produkt gewaschen, vorzugsweise mittels C₁-C₄-Alkohol und/oder Wasser.

Das erfindungsgemäße Verfahren wird vorzugsweise wie folgt durchgeführt:
Die Verbindung der Formel (III), vorzugsweise Dibenzylamin, und ein Alkohol, vorzugsweise Isopropanol werden in Wasser vorgelegt. Nach Rühren der Reaktionsmischung wird vorzugsweise bei Temperaturen von 40 bis 50°C eine Mischung aus der Verbindung der Formel (II), vorzugsweise Natrium-Mercaptobenzothiazol (NaMBT), und Natriumhypochlorit zugegeben. Der pH-Wert während der Zudosierung liegt vorzugsweise im Bereich von 10,3 bis 11, welcher idealerweise mittels Schwefelsäure eingestellt wird.

Anschließend wird vorzugsweise mittels Natronlauge ein pH-Wert im Bereich von 11,3 bis 12 eingestellt, nachgerührt und vorzugsweise Wasser zugesetzt. Das Produkt wird vorzugsweise abfiltriert. Die anschließende Reinigung erfolgt vorzugsweise durch Waschen zunächst mit Alkohol, vorzugsweise Isopropanol bei 35 - 45°C und anschließend, vorzugsweise mit Wasser, bei 55 - 65°C.

Die vorliegende Erfindung wird durch das nachfolgenden Versuchsbeispiel näher erläutert ohne, dass die Erfindung auf dieses beschränkt ist.

### Ausführungsbeispiele

### Beispiel 1

Als Apparatur wurde ein 2l Planschliffbecher, ausgestattet mit Mig-Rührer (3 Blatt); Heizung/Kühlung über Mantel; pH- und Redoxmessungsvorrichtung; Thermometer; Dosierpumpen für Natriumhypochlorit (NaOCl) und die wässrige Lösung das Natriumsalzes des 2-Mercaptobenzothiazols (NaMBT) sowie einen Tropftrichter für eine pH-Wertgeregelte Dosierung von Schwefelsäure bzw. Natronlauge eingesetzt.

In diesem Planschliffbecher wurden 203,4 g Dibenzylamin (97%ig) und 531 g Isopropanol (Wassergehalt < 1%) mit 195g Wasser vorgelegt. Anschließend rührte man die Reaktionsmischung und innerhalb von 3 Stunden wurde bei einer Innentemperatur von 46°C folgende Mischung zudosiert:
446,1 g 50,50%ige wässrige NaMBT-Lösung (Dichte: 1,245) = 119 ml/h (358 ml)
834,2 g 14,21%ige wässrige NaOCl (Dichte: 1,22) = 228 ml/h (683,8 ml)

Dann wurde zu den im Planschliffbecher vorlegten Chemikalien innerhalb von 1- 5 bis Minuten 5 g NaOCl hinzudosiert.

Während der Dosierung wurde im Reaktionsgemisch ein pH-Wert von 10,5 eingehalten, was mittels Schwefelsäure erfolgte.

Anschließend wurde mit 1,6 g 48%iger Natronlauge ein pH von 11,5 eingestellt, 245 ml Wasser zugesetzt und 15 Minuten gerührt. Die Suspension wurde bei 40°C über Schwarzbandfilter ø 18,5 cm abgenutscht.

Das Produkt wurde abfiltriert, mit 625 g wässrigem Isopropanol (35%ig) bei 40°C und anschließend mit 1165 g Wasser bei 60°C gewaschen.

Der Filterkuchen wurde bis zur Massenkonstanz im Trockenschrank bei ca. 50°C getrocknet.

Man erhielt 283,6 g N,N-Dibenzyl-benzthiazolylsulfenamid (DBBS). Laut HPLC-Analyse lag der Wirkstoffgehalt (Titration) bei über 98 %; freies Amin wurde mit < 0,7% gemessen.

Des Weiteren lag der Schmelzpunkt in einem Bereich von 90 - 100°C, vorzugsweise von 92,5 - 93,2°C.

Es zeigte sich, dass durch das erfindungsgemäße Verfahren die Herstellung von N,N-substituierten Benzthiazolyl-sulfenamiden der Formel (I) gelang, ohne dass der Einsatz von elementarem Chlor notwendig wurde.

## Patentansprüche

1. Verfahren zur Herstellung von N,N-disubstituierten Benzthiazolyl-sulfenamiden der Formel (I) 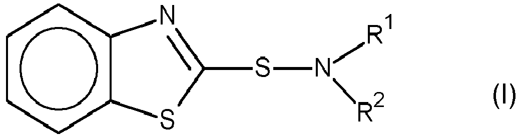
mit R¹, R² = unabhängig voneinander -CH(R³)(R⁴)
mit R³, R⁴ = unabhängig voneinander H, C₁-C₃-Alkyl, vorzugsweise Methyl, 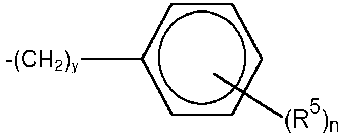
und R⁵ = C₁-C₃-Alkyl,
y = 0 bis 6, vorzugsweise 0 bis 2, besonders bevorzugt 0 und
n = 0 bis 5, vorzugsweise 0,
durch Umsetzung von 2-Mercaptobenzthiazolsalzen der Formel (II) 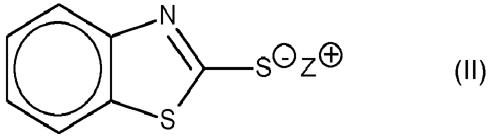 mit Z = Na oder K, vorzugsweise Na,
mit Aminen der Formel (III)
NH(R¹)(R²) (III)
worin R¹ und R² die obige Bedeutung haben,
**dadurch gekennzeichnet, dass** die Umsetzung in Gegenwart von Natriumhypochlorit durchgeführt wird.

2. Verfahren nach Anspruch 1, **dadurch gekennzeichnet, dass** es sich bei den N,N-substituierten Benzthiazolylsulfenamiden der Formel (I) um N,N-Dibenzylbenzthiazolylsulfenamid (DBBS) oder N,N-Diisopropylbenzthiazolyl-sulfenamid handelt.

3. Verfahren nach Anspruch 1 oder 2, **dadurch gekennzeichnet, dass** als Verbindung der Formel (II) 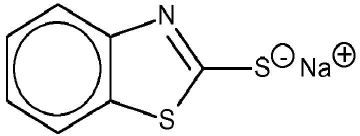 und
als Verbindung der Formel (III) 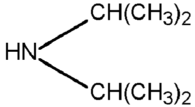 oder 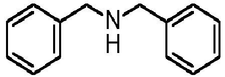 eingesetzt wird.

4. Verfahren nach einem der Ansprüche 1 bis 3, **dadurch gekennzeichnet, dass** diese Umsetzung bei Temperaturen von 30 bis 50 °C durchgeführt wird.

5. Verfahren nach einem der Ansprüche 1 bis 4, **dadurch gekennzeichnet, dass** diese Umsetzung bei pH-Werten von 10 bis 12 durchgeführt wird.

6. Verfahren nach einem der Ansprüche 1 bis 5, **dadurch gekennzeichnet, dass** diese Umsetzung in Gegenwart von mindestens einem C₁-C₄-Alkohol und/oder Wasser durchgeführt wird.

7. Verfahren nach Anspruch 5, **dadurch gekennzeichnet, dass** die Verbindung der Formel (III) gegebenenfalls in Anwesenheit von Wasser und/oder Alkohol vorgelegt und die Verbindung der Formel (II) und Natriumhypochlorit zudosiert werden, wobei der pH-Wert während der Zudosierung mit Schwefelsäure eingestellt wird.

8. Verfahren nach einem der Ansprüche 5 oder 7, **dadurch gekennzeichnet** das der pH-Wert nach Zugabe der Verbindung der Formel (II) und dem Natriumhypochlorit mit Natronlauge eingestellt wird.

9. Verfahren nach einem der Ansprüche 1 bis 7, **dadurch gekennzeichnet, dass** die die Verbindung der Formel (II) zu der Verbindung der Formel (III) in einem molaren Verhältnis von 1,4 : 1 bis 0,8 : 1 eingesetzt wird.
